# EUROPÄISCHE PATENTSCHRIFT

(11) **EP 2 621 878 B1**
(45) Veröffentlichungstag und Bekanntmachung des Hinweises auf die Patenterteilung: **01.06.2016**
(21) Anmeldenummer: 11766943.2
(22) Anmeldetag: 28.09.2011
(51) Int. Cl.: C07C 45/80, C07C 47/127

(54) **VERFAHREN ZUR REINIGUNG WÄSSRIGER GLYOXAL-LÖSUNGEN**
PROCESS FOR PURIFYING AQUEOUS GLYOXAL SOLUTIONS
PROCÉDÉ DE PURIFICATION DE SOLUTIONS AQUEUSES DE GLYOXAL

(30) Priorität: 01.10.2010 EP 10186019
(43) Veröffentlichungstag der Anmeldung: 07.08.2013
(73) Patentinhaber: BASF SE, 67056 Ludwigshafen am Rhein (DE)
(72) Erfinder: DECKERT, Petra, 69245 Bammental (DE); GROLL, Peter, 67125 Dannstadt-Schauernheim (DE); RUMPF, Bernd, 68766 Hockenheim (DE); HORN, Christian, 67308 Rüssingen (DE)
(74) Vertreter: BASF IP Association
(86) Internationale Anmeldenummer: PCT/EP2011/066812
(87) Internationale Veröffentlichungsnummer: WO 2012/041875

(56) Entgegenhaltungen:
- WO-A2-2010/076252
- DE-A1- 3 402 733

## Beschreibung

Die vorliegende Erfindung betrifft ein Verfahren zur Reinigung von wässrigen Glyoxal-Lösungen, die mindestens eine Säure enthalten, mit einer lonentauscher-Lösung sowie die nach diesem Verfahren erhältlichen wässrigen Glyoxal-Lösungen.

Glyoxal wird beispielsweise als Hilfskomponente in der Textil- oder Papierindustrie verwendet. Glyoxal wird üblicherweise durch Oxidation von Acetaldehyd oder durch Oxidehydrierung des entsprechenden Glykols an einem katalytischen Festbett hergestellt. Als Katalysator kommt dabei beispielsweise mit Phosphor dotiertes Kupfer zum Einsatz. Die bei diesem Verfahren anfallenden wässrigen Glyoxal-Lösungen weisen dabei Nebenprodukte auf, die vor ihrer weiteren Verwendung abgetrennt werden müssen. Typische Nebenprodukte sind dabei Formaldehyd, Glykolaldehyd, Ameisensäure, Essigsäure, sowie schwerflüchtige Säuren wie Glyoxylsäure, Glykolsäure und Oxalsäure. Das durch Oxidehydrierung aus Glykol hergestellte Glyoxal enthält in der Regel nicht mehr als 2 Gew.-% Säure. Für den kommerziellen Einsatz des Glyoxals werden jedoch Säurezahlen von < 5 mg KOH/g gefordert.

Zur Abtrennung der oben genannten Säuren, vor allem der schwerflüchtigen Säuren, sind im Stand der Technik verschiedene Verfahren beschrieben. US 3 270 062 beschreibt ein Verfahren zur Reinigung von wässrigen Glyoxal-Lösungen durch Behandlung der Glyoxal-Lösung mit einem festen Ionenaustauscher. Dieses Verfahren hat den Nachteil einer diskontinuierlichen Arbeitsweise. Außerdem müssen die lonentauscher wegen der hohen Säurewerte der eingesetzten wässrigen Glyoxal-Lösungen häufig regeneriert werden. Aus diesem Grund fallen bei diesem Verfahren erhebliche Mengen verdünnter Glyoxal-Lösungen an, so dass das in US 3 270 062 beschriebene Verfahren nicht wirtschaftlich betrieben werden kann.

Es ist weiterhin bekannt, zur Entfernung von sauren Verunreinigungen aus wässrigen Glyoxal-Lösungen diese mit einer Lösung hochmolekularer tertiärer Amine oder quaternärer Ammoniumsalze in Bicarbonatform in einem organischen Lösungsmittel zu behandeln. Bei diesem Verfahren führt man die beiden Lösungen in einer vielstufigen Extraktionskolonne zueinander im Gegenstrom. Dadurch wird zwar eine kontinuierliche Reinigung erreicht, es sind jedoch lange Verweilzeiten erforderlich. Außerdem ist bei diesem Verfahren zur Vermeidung von zu großen Glyoxalverlusten eine Reextraktion der organischen Phase mit Wasser notwendig. Dies führt dazu, dass dieses Verfahren unattraktiv ist.

Eine Aufarbeitung des durch Oxidation von Acetaldehyd oder durch Oxidehydrierung hergestellten Glyoxals ist zwingend notwendig, da die nach diesem Verfahren erhaltenen Glyoxal-Lösungen eine starke Gelbfärbung aufweisen. Für die kommerziell eingesetzten Glyoxal-Lösungen werden im Endprodukt typischerweise Farbzahlen von < 200 Apha gefordert.

WO 2010/076252 (D1) offenbart ein Verfahren zur Reinigung einer wässrigen Glyoxal-Lösung, die mindestens eine Säure enthält, durch extraktive Säureabtrennung umfassend die Schritte:
I) Vermischen einer wässrigen Glyoxal-Lösung mit einer lonentauscher-Lösung, enthaltend 20 bis 60 Gew.-% eines tertiären Amins und 80 bis 40 Gew.-% eines mit Wasser nicht beliebig mischbaren organischen Lösungsmittels, bei einer Verweilzeit von < 5 Minuten und einer Temperatur von 30 bis 100 °C,
II) Auftrennung der Mischung aus Schritt I) in eine extrahierte wässrige Glyoxal-Lösung und eine beladene lonentauscher-Lösung und Abtrennung der beladenen lonentauscher-Lösung bei Temperaturen von 30 bis 100 °C, und
III) Auftrennung der extrahierten wässrigen Glyoxal-Lösung in tertiäres Amin und gereinigte wässrige Glyoxal-Lösung und Abtrennung des tertiären Amins bei Temperaturen < 30 °C.

DE 34 02 733 beschreibt ein Verfahren zur Reinigung wässriger Glyoxal-Lösungen durch Extraktion der in der Gyoxal-Lösung enthaltenen Säuren mit einer Lösung eines tertiären Amins in einem organischen Lösungsmitten.

Bei dem dort beschriebenen Verfahren werden wässrige Glyoxal-Lösungen erhalten, die eine Säurezahl < 5 mg KOH/g und eine niedrige Farbzahl aufweisen. Die nach diesem Verfahren erhältlichen Glyoxal-Lösungen entsprechen somit Spezifikationen, die für kommerzielle Glyoxal-Lösungen gefordert werden. Bei dem in DE 34 02 733 beschriebenen Verfahren ist zudem der Glyoxal-Verlust in die Lösung aus tertiärem Amin und organischem Lösungsmittel gering.

Die Regeneration der lonentauscher-Lösung erfolgt in an sich bekannter Weise durch inkontaktbringen mit basischen Verbindungen wie Natriumhydroxid, Kaliumhydroxid und Natriumbicarbonat und Neutralisation der in der lonentauscher-Lösung gebundenen Säure. Typischer Weise enthält die neutralisierte lonentauscher-Lösung noch beträchtliche Mengen an bei der Neutralisation gebildeten Salzen, welche bei der Säureextraktion in die Glyoxal-Lösung übergehen würden und die Produktqualität beeinträchtigen würden. Daher wird, bevor der regenerierte lonentauscher wieder als Extraktionsmittel eingesetzt wird, eine Salzwäsche mit Wasser durchgeführt.

Ein Problem bei der Säureextraktion ist der Glyoxalverlust durch in die Extraktionsmittelphase übergehendes Glyoxal. Dieses wird bei der Regeneration der lonentauscher-Lösung zum Teil durch Canizarro-Reaktion abgebaut, die Abbauprodukte gelangen in das Abwasser.

Aufgabe der vorliegenden Erfindung ist es, ein Verfahren zur Reinigung von wässriger Glyoxal-Lösung bereitzustellen, das durch einen niedrigen Glyoxalverlust gekennzeichnet ist.

Gelöst wird die Aufgabe durch ein Verfahren zur Reinigung einer wässrigen GlyoxalLösung, die mindestens eine Säure enthält, durch extraktive Säureabtrennung, umfassend die Schritte
i) Vermischen und Dispergieren der wässrigen Glyoxal-Lösung mit einer Ionentauscher-Lösung, enthaltend 20 bis 60 Gew.-% eines tertiären Amins und 80 bis 40 Gew.-% eines mit Wasser nicht beliebig mischbaren organischen Lösungsmittels. bei einer Temperatur von 30 bis 100°C,
ii) Phasentrennung und Abtrennung der mit der Säure beladenen Ionentauscher-Lösung von der wässrigen Glyoxal-Lösung bei Temperaturen von 30 bis 100°C, und
iii) Regeneration der lonentauscher-Lösung durch Inkontaktbringen mit einer basischen Verbindung zur Neutralisation der Säure und Abtrennung der basischen Verbindung,
iv) Vermischen und Dispergieren der Ionentauscher-Lösung mit Wasser zur Reinigung von bei der Neutralisation gebildeten Salzen, wobei eine Dispersion aus Wasser und lonentauscher-Lösung erhalten wird,
v) Phasentrennung und Abtrennung der wässrigen Phase von der Ionentauscher-Lösung, und Rückführung der lonentauscher-Lösung in Schritt i),
dadurch gekennzeichnet, dass Phasentrennung und Abtrennung der wässrigen Phase in Schritt v) derart erfolgen, dass der Gehalt der wässrigen Phase der in Schritt v) erhaltenen regenerierten lonentauscher-Lösung < 1 Gew.-% beträgt.

Der Gehalt der wässrigen Phase ist die Menge der in der lonentauscher-Lösung verbliebenen Menge an (prinzipiell abtrennbarer) nicht-gelöster Fremdphase und schließt die Menge an in der lonentauscher-Lösung homogen gelöstem Wasser nicht ein.

Es wurde überraschender Weise gefunden, dass durch einen möglichst geringen Wassergehalt der nach der Regeneration erhaltenen lonentauscher-Lösung der Glyoxal-Verlust im nachfolgenden Säureextraktionsschritt minimiert werden kann.

In Schritt i) des erfindungsgemäßen Verfahrens werden wässrige Glyoxal-Lösungen, die herstellungsbedingt mindestens eine Säure als Nebenprodukt enthalten, mit einer Ionen-tauscher-Lösung vermischt. Wässrige Glyoxal-Lösungen werden im Allgemeinen durch Oxidation von Acetaldehyd oder durch Oxidehydrierung von Monoethylenglykol an einem Katalysator erhalten. Beispielsweise werden wässrige Glyoxal-Lösungen erfindungsgemäß behandelt, die entsprechend dem in DE 19 23 048 beschriebenen Verfahren durch Oxidehydrierung an einem Katalysatorfestbett enthaltend Phosphor dotiertes Kupfer als Katalysator zugänglich sind.

Die wässrige Glyoxal-Lösung kann auch nach den in DE 500 04 079 und EP 1 169 119 beschriebenen Verfahren erhalten werden. Dazu wird Monoethylenglykol in einem geeigneten Verdampfer in die Gasphase überführt und in Gegenwart von Sauerstoff in einem Reaktor dehydriert. Das den Reaktor verlassende gasförmige Produktgemisch, welches neben Glyoxal andere Nebenprodukte enthält, wird abgekühlt. Dabei werden die kondensierbaren Komponenten, wie Wasser und Glyoxal, sowie Nebenprodukte wie Formaldehyd, Glykolaldehyd, Ameisensäure, sowie schwerflüchtige Säuren wie Glyoxylsäure, Glykolsäure und Oxalsäure, auskondensiert. Die weitere Aufarbeitung des Produktstroms erfolgt durch Abstrippung leicht siedender Komponenten, wie Formaldehyd, Ameisensäure und Essigsäure mittels Wasserdampf. Die so erhaltenen wässrigen Glyoxal-Lösungen enthalten noch schwerflüchtige Säuren wie Glyoxylsäure, Glykolsäure und Oxalsäure. Auch die nach den Verfahren gemäß DE 500 04 079 und EP 1 169 119 zugänglichen Glyoxal-Lösungen sind zum Einsatz in dem erfindungsgemäßen Verfahren besonders geeignet.

Die in Schritt i) eingesetzten wässrigen Glyoxal-Lösungen enthalten also mindestens eine Säure, ausgewählt aus der Gruppe bestehend aus Ameisensäure, Essigsäure, Glyoxylsäure, Glykolsäure und Oxalsäure oder Mischungen aus zwei oder mehr der vorstehend genannten Säuren, in einer Konzentration im Bereich von 0,2 bis 2 Gew.-%, bezogen auf das Gesamtgewicht der wässrigen Glyoxal-Lösung.

Die Säurezahlen der in dem erfindungsgemäßen Verfahren eingesetzten wässrigen Glyoxal-Lösungen können in weiten Bereichen variieren. Bevorzugt sind jedoch wässrige Glyoxal-Lösungen, die Säurezahlen ≤ 100 mg KOH/g, bevorzugt ≤ 50 mg KOH/g und besonders bevorzugt ≤ 30 mg KOH/g aufweisen.

Auch der Glyoxalgehalt der in dem erfindungsgemäßen Verfahren eingesetzten wässrigen Glyoxal-Lösungen ist unkritisch und kann in weiten Bereichen variieren, Im Allgemeinen werden jedoch wässrige Glyoxal-Lösungen mit einem Glyoxalgehalt ≤ 55 Gew.-%, bevorzugt ≤ 50 Gew.-% und besonders bevorzugt ≤ 45 Gew.-% eingesetzt, wobei Gew.-% jeweils auf das Gesamtgewicht der eingesetzten wässrigen Glyoxal-Lösung bezogen sind. Der Glyoxalgehalt der in dem erfindungsgemäßen Verfahren eingesetzten Lösungen beträgt im Allgemeinen mindestens 30 Gew.-%, bevorzugt mindestens 35 Gew.-%, besonders bevorzugt mindestens 38 Gew.-%. Beispielsweise beträgt die Glyoxalkonzentration der eingesetzten Lösung 38 bis 45%.

Die wässrige Glyoxal-Lösung wird in Schritt i) mit einer lonentauscher-Lösung vermischt. Unter einer lonentauscher-Lösung im Rahmen der vorliegenden Erfindung wird ein Gemisch, das mindestens ein tertiäres Amin und ein mit Wasser nicht beliebig mischbares organisches Lösungsmittel enthält, verstanden.

Geeignete tertiäre Amine sind geradkettige oder verzweigte aliphatische Amine mit Molekulargewichten von 300 bis 600 g/mol. Bevorzugte tertiäre Amine sind Trioctylamin, Trinonylamin, Tridecylamin und Tridodecylamin. Es ist auch möglich, Mischungen aus zwei oder mehr der bevorzugten tertiären Amine einzusetzen. Darüber hinaus können auch ein oder mehrere der bevorzugten tertiären Amine im Gemisch mit einem oder mehreren weiteren Aminen eingesetzt werden. Besonders bevorzugt sind Mischungen aus zwei tertiären Aminen, ausgewählt aus Trioctylamin, Trinonylamin, Tridecylamin und Tridodecylamin. Insbesondere bevorzugt sind Mischungen aus Trioctylamin und Tridecylamin.

Als mit Wasser nicht beliebig mischbare organische Lösungsmittel kommen alle organischen Lösungsmittel in Betracht, die mit Wasser unter Normalbedingungen nicht in jedem Verhältnis eine echte Lösung ausbilden und mit Glyoxal unter den bei der Extraktion herrschenden Bedingungen nicht reagieren. Bevorzugte organische Lösungsmittel sind mit Wasser nicht in beliebigen Verhältnissen mischbare geradkettige oder verzweigte primäre, sekundäre oder tertiäre Alkohole mit mehr als 3 C-Atomen. Bevorzugt sind aliphatische Alkohole mit 3 bis 15 C-Atomen, besonders bevorzugt sind aliphatische Alkohole mit 8 bis 13 C-Atomen. Insbesondere bevorzugt sind Pentanol, Hexanol, 2-Ethylhexanol, Octanol, Decanol und Isodecanol. Als mit Wasser nicht beliebig mischbare organische Lösungsmittel können auch Mischungen der vorstehend genannten Lösungsmittel eingesetzt werden.

Die lonentauscher-Lösung enthält im Allgemeinen 20 bis 60 Gew.-%, bevorzugt 30 bis 50 Gew.-% mindestens eines tertiären Amins und 80 bis 40 Gew.-% bevorzugt 70 bis 50 Gew.-% eines mit Wasser nicht beliebig mischbaren organischen Lösungsmittels, jeweils bezogen auf das Gesamtgewicht der lonentauscher-Lösung.

In Schritt i) des erfindungsgemäßen Verfahrens wird die wässrige Glyoxal-Lösung mit der lonentauscher-Lösung vermischt. Das Vermischen erfolgt dabei bei Temperaturen von 30 bis 100°C, bevorzugt von 30 bis 80°C und besonders bevorzugt von 30 bis 60°C. Das Volumenverhältnis von wässriger Glyoxal-Lösung und lonentauscher-Lösung ist frei wählbar. Bevorzugt sind Volumenverhältnisse zwischen 10:1 und 1:3.

Das Vermischen der wässrigen Glyoxal-Lösung mit der Ionentauscher-Lösung kann dabei in jedem bekannten Mischapparat erfolgen. Es hat sich gezeigt, dass die Verweilzeit der beiden Lösungen möglichst kurz sein sollte, so dass sich die Gleichgewichtskonzentration von Glyoxal in der lonentauscher-Phase nicht einstellen kann. Unter Verweilzeit wird die Zeitspanne verstanden, während der sich die wässrige Glyoxal-Lösung und die Ionentauscher-Lösung im Mischapparat in Durchmischung befinden. In Durchmischung befinden sich die Phasen im Wesentlichen solange, wie der Misch- und Dispergiervorgang aufrechterhalten wird. Durch eine möglichst kurze Verweilzeit kann der Übergang von Glyoxal aus der wässrigen Glyoxal-Lösung in die lonentauscher-Lösung und damit der Verlust von Glyoxal möglichst gering gehalten werden. Geeignete Verweilzeiten liegen unter 5 Minuten, bevorzugt im Bereich von 1 Sekunde bis 2 Minuten, insbesondere bevorzugt im Bereich von 1 sec bis 20 sec.

Dabei werden die in der wässrigen Glyoxal-Lösung enthaltenen Säuren an die in der Io-nentauscher-Lösung enthaltenen tertiären Amine gebunden. Dabei entsteht eine mit der gebundenen Säure beladene lonentauscher-Lösung, die in der Glyoxal-Lösung enthaltene Säuren gebunden enthält. Geeignete Mischapparate ermöglichen eine möglichst rückvermischungsfreie Dispergierung bei sehr kurzer Verweilzeit, wobei gleichzeitig eine gute Phasentrennung ermöglicht wird. Die Vermischung der beiden Lösungen erfolgt dabei bevorzugt einstufig durch das gleichzeitige Einleiten von wässriger Glyoxal-Lösung und Ionentauscher-Lösung in den Mischapparat. In einer bevorzugten Ausführungsform wird hierzu ein statischer Mischer eingesetzt. In einer weiteren bevorzugten Ausführungsform wird ein In-Line-Mischer oder ein Lochblenden-Mischer eingesetzt.

In Schritt ii) erfolgt die Phasentrennung in eine extrahierte, von Säuren weitgehend befreite wässrige Glyoxal-Lösung und die mit der Säure beladene lonentauscher-Lösung. Die Auftrennung und Abtrennung kann in jeder beliebigen Trennapparatur, wie Phasenscheider, Zentrifuge oder Phasenscheider in Kombination mit Koaleszierfilter erfolgen.

Der Koaleszierfilter kann dabei dem Phasenscheider vorgeschaltet oder im Phasenscheider integriert sein. Der Phasenscheider kann als Trennhilfen Einbauten wie Gestricke, Füll-körper und/oder Platten enthalten. Die Phasentrennung wird dabei bei Temperaturen von 30 bis 100°C, bevorzugt bei 30 bis 80°C und besonders bevorzugt bei 30 bis 60°C vorgenommen.

In einem weiteren Schritt kann die in Schritt ii) erhaltene extrahierte wässrige Glyoxal-Lösung noch weiter aufgereinigt werden. Es hat sich gezeigt, dass die vorstehend beschriebene Abtrennung der lonentauscher-Lösung in Schritt ii) nicht immer vollständig ist und dass geringe Mengen an tertiärem Amin und des Lösungsmittels aus der Ionentauscher-Lösung in der extrahierten wässrigen Glyoxal-Lösung verbleiben.

Zur Abtrennung des Restgehalts an tertiärem Amin und Lösungsmittel wird die extrahierte wässrige Glyoxal-Lösung aus Schritt ii) auf Temperaturen < 30°C, bevorzugt auf Temperaturen im Bereich von 10 bis 25°C abgekühlt. Dadurch kommt es zu einer weiteren Abscheidung von tertiärem Amin und gegebenenfalls von organischem Lösungsmittel.

Gegebenenfalls werden weitere Nebenprodukte aus der wässrigen Glyoxal-Lösung im Anschluss an die Säureextraktion mittels eines Adsorbers entfernt.

Die Abtrennung des abgeschiedenen tertiären Amins kann durch jedes bekannte Trennverfahren erfolgen. Geeignete Trennverfahren wurden vorstehend beschrieben. Bevorzugt ist die Abtrennung durch einen Koaleszierfilter mit anschließendem Phasenscheider oder durch einen Phasenscheider mit integriertem Koaleszierfilter oder durch eine Zentrifuge.

Insbesondere bevorzugt wird Schritt ii) mit einem Koaleszierfilter mit anschließendem Phasenscheider oder mit einem Phasenscheider mit integriertem Koaleszierfilter durchgeführt. Dabei wird die Phasentrennung durch Durchleiten der Dispersion durch den Koaleszierfilter verbessert. Bevorzugte Materialien für den Koaleszierfilter sind Kunststoffe, insbesondere Polypropylen und Polyethylen oder Mischungen daraus.

In Schritt iii) wird die lonentauscher-Lösung durch Inkontaktbringen mit einer basischen Verbindung zur Neutralisation der gebundenen Säure regeneriert.

Geeignete basische Verbindungen sind beispielsweise Natriumhydroxid, Kaliumhydroxid und Natriumbicarbonat. Diese werden im Allgemeinen in Form ihrer wässrigen Lösungen zugegeben. Im Allgemeinen enthalten diese 1 bis 30 Gew.-%, bevorzugt 2 bis 15 Gew.-%, beispielsweise 5 Gew.-% der basischen Verbindung.

Dabei wird die Lösung der basischen Verbindung zugegeben und mit der Ionentauscher-Lösung in einem geeigneten Mischaggregat und bei einer Temperatur von 30 bis 100 °C dispergiert. Im Allgemeinen beträgt das Gewichtsverhältnis Lösung der basischen Verbindung : lonentauscher-Lösung 0,02 : 1 bis 1 : 1. Dabei bildet die Lösung der basischen Verbindung im Allgemeinen die disperse Phase. Die Verweilzeit beträgt im Allgemeinen < 20 min, bevorzugt beträgt sie 0,1 bis 15 min, besonders bevorzugt 2 bis 8 min.

Geeignete Dispergieraggregate sind z. B. eine Mischpumpe, ein statischer Mischer oder ein Rührbehälter.

In Schritt iv) wird die lonentauscher-Lösung mit Wasser zur Reinigung von bei der Neutralisation gebildeten Salzen vermischt.

Dabei wird Wasser zugegeben und mit der lonentauscher-Lösung in einem geeigneten Mischaggregat und bei einer Temperatur von 30 bis 100 °C dispergiert. Im Allgemeinen beträgt das Gewichtsverhältnis Wasser: lonentauscher-Lösung 0,1 : 1 bis 1 : 1. Dabei bildet Wasser die disperse Phase. Die Verweilzeit beträgt im Allgemeinen < 20 min, bevorzugt beträgt sie 0,1 bis 15 min, besonders bevorzugt 2 bis 8 min.

Geeignete Dispergieraggregate sind ebenfalls z. B. eine Mischpumpe, ein statischer Mischer oder ein Rührbehälter.

In Schritt v) erfolgt die Phasentrennung und Abtrennung der wässrigen Phase von der Ionentauscher-Lösung. Die abgetrennte lonentauscher-Lösung wird anschließend wieder in den Säureextraktionsschritt i) zurückgeführt.

Wesentliches Merkmal der Erfindung ist, dass die Phasentrennung und die Abtrennung der die gelösten Salze enthaltenden wässrigen Phase in Schritt v) derart erfolgen, dass der Gehalt der wässrigen Phase der in Schritt v) erhaltenen regenerierten Ionentauscher-Lösung < 1 Gew.-% beträgt. Bevorzugt beträgt der Gehalt der wässrigen Phase < 0,5 Gew.-%, besonders bevorzugt < 0,2 Gew.-%, insbesondere < 0,1 Gew.-%.

Der Gehalt der wässrigen Fremdphase wird ermittelt durch Bestimmung des Gesamtwas-sergehaltes der lonentauscher-Lösung und der Konzentration von in der Ionentauscher-Lösung homogen gelöstem Wasser, jeweils nach der Karl-Fischer-Methode.

Die Auftrennung der Dispersion aus Schritt iv) und Abtrennung der wässrigen Phase kann in einer wirksamen Trennapparatur, wie einem Phasenscheider mit trennwirksamen Einbauten wie Gestricken oder Platten oder in einem Phasenscheider in Kombination mit einem vorgeschalteten Koaleszierfilter, in einer Zentrifuge, einem Hydrozyklon oder Elektro-abscheider erfolgen. Der Koaleszierfilter kann dabei ein dem Phasenscheider vorgeschalteteter separater Apparat sein oder in den Phasenscheider integriertert sein. Durch Durchleiten der Dispersion durch den Koaleszierfilter wird dabei eine verbesserte Phasentrennung bewirkt. Geeignete Materialien für den Koaleszierfilter sind alle Materialien, die gegenüber der lonentauscher-Lösung beständig sind. Geeignete Materialien sind beispielsweise Fasern aus Phenolharz, Glas, Metall oder Baumwolle. Der Dispergierschritt iv) und der Auftrennschritt v) können auch gemeinsam in einer Extraktionskolonne durchgeführt werden.

Die Erfindung wird durch die Beispiele näher erläutert.

### Beispiele:

### Beispiel 1 Einfluss des Glyoxal-Gehaltes im Zulauf zur Säureextraktion auf die Löslichkeit von Glyoxal in der lonentauscher-Lösung

In einem Rührgefäß wurde die lonentauscher-Lösung, bestehend aus 40 % Hostarex A327 (Mischung aus 50 % Tridodecylamin und 50 % Trioctylamin der Firma Clariant) und 60 % Isodekanol bei 45°C mit säurehaltigen Glyoxal-Lösungen aus einem Produktionsbetrieb dispergiert, wobei jeweils 0,5 kg lonentauscher-Lösung bezogen auf 1 kg Glyoxal-Lösung zugesetzt wurden. Die Versuche wurden mit Glyoxal-Lösungen durchgeführt, die unterschiedliche Glyoxal-Gehalte zwischen 30 und 42 Gew.-% aufwiesen. Nach 10 min Rührzeit bei konstanter Drehzahl wurden dem Rührgefäß Proben entnommen, und nach der Phasentrennung wurden die Glyoxal-Konzentrationen in den klaren Glyoxal-Phasen analysiert.

**Tabelle 1**

| Glyoxal-Konzentration vor Extraktion [Gew.-%] | Glyoxal-Konzentration der extrahierten Glyoxal-Phase [Gew.-%] | Verringerung der Glyoxal-Konzentration [Gew.-%] | Glyoxalverlust [%] |
|---|---|---|---|
| 41,1 | 38,0 | 3,5 | 8,4 |
| 37,8 | 34,8 | 3,1 | 8,1 |
| 30,4 | 28,0 | 2,4 | 8,0 |

Die Gleichgewichts-Konzentrationen an Glyoxal in der lonentauscher-Lösung sind höher, wenn eine höher konzentrierte Glyoxal-Lösung extrahiert wird. D.h. je höher die Glyoxal-Konzentration im Zulauf zur Extraktion, desto höher ist die Reduzierung der Glyoxal-Konzentration im Gleichgewicht und damit der Verlust.

### Beispiele 2 und 3 Einfluss von wässriger Fremdphase in der lonentauscher-Lösung auf den Glyoxal-Verlust in der Säureextraktion bei kurzen Dispergierzeiten

In einem Rührgefäß wurde die lonentauscher-Lösung, bestehend aus 40 % Hostarex A327 (Mischung aus 50 % Tridodecylamin und 50 % Trioctylamin der Firma Clariant) und 60 % Isodekanol bei 45°C mit säurehaltigen Glyoxal-Lösungen aus einem Produktionsbetrieb dispergiert, wobei jeweils 0,5 kg lonentauscher-Lösung bezogen auf 1 kg Glyoxal-Lösung zugesetzt wurden. Bei den Versuchen wurden 5 Gew.-% Wasser bezogen auf die Ionentauscher-Phase zusätzlich zugegeben. Bei einem Versuch wurde das Wasser vor der Dispergierung der Glyoxal-Phase zugegeben, wodurch die Feedkonzentration von 41,6 auf 40,4 Gew.-% Glyoxal abnahm. Beim zweiten Versuch wurde die gleiche Wassermenge mit der lonentauscher-Phase dispergiert, bevor die wässrige Glyoxal-Phase zugegeben wurde. Nach 0,5 und 2 min Rührzeit bei konstanter Drehzahl wurden dem Rührgefäß Proben der Dispersion entnommen, und nach der sofortigen Phasentrennung durch Zentrifugieren wurden die Säurezahlen mittels Titration und Glyoxal-Konzentrationen in den klaren Glyoxal-Phasen analysiert. Die Daten sind in der folgenden Tabelle 2 aufgeführt.

**Tabelle 2**

| | Zugabe von 5 Gew.-% Wasser vor der Dispergierung zur | Rührzeit [min] | Säurezahl in der Glyoxal-Phase [mg KOH/g] | Glyoxal-Konzentration [Gew.-%] | Glyoxalverlust [%] |
|---|---|---|---|---|---|
| Beispiel 2 | Glyoxal-Lösung | 0,5 | 0,52 | 40,0 | 1,0 |
| | | 2 | 0,31 | 39,6 | 1,9 |
| Beispiel 3 | lonentauscherlösung | 0,5 | 0,35 | 39,9 | 1,3 |
| | | 2 | 0,36 | 39,2 | 2,9 |

Die Säuren wurden schnell extrahiert. Säurezahlen kleiner 1 mg KOH/g wurden bereits bei Dispergierzeiten von 0,5 min. erreicht. Die Glyoxal-Verluste nach einer Dispergierzeit von 0,5 min waren deutlich geringer als im Gleichgewicht bei einer Dispergierzeit von 10 min.

Wurde das zusätzliche Wasser vor dem Dispergieren von Glyoxal-Lösung und Ionentauscher-Lösung in der Ionentauscher-Lösung dispergiert, so stieg der Verlust an Glyoxal an, weil durch die anfänglich höhere Konzentration an Glyoxal in der Glyoxal-Lösung mehr Glyoxal in die lonentauscher-Lösung extrahiert wurde. In diesem Fall dauert auch die Grobtrennung der beiden Phasen mit >105 min länger, als wenn das zusätzliche Wasser zur Glyoxal-Lösung zugegeben wurde (Grobtrennzeit 45 min). D.h., dass eine ungenügende Phasentrennung bei der Wäsche der neutralisierten lonentauscher-Lösung zu einer Verschlechterung der Phasentrennung in der Säureextraktion führt, wodurch der Glyoxal-Verlust ansteigt.

### Beispiel 4 Phasentrennung von regenerierter lonentauscher-Lösung und Waschabwasser mittels Koaleszierfilter

In einem Rührgefäß wurde neutralisierte lonentauscher-Lösung aus einem Produktionsbetrieb, bestehend aus 40 % Hostarex A327 (Mischung aus 50 % Tridodecylamin und 50 % Trioctylamin der Firma Clariant) und 60 % Isodekanol bei 45°C mit VE-Wasser dispergiert, wobei 0,4 kg VE-Wasser bezogen auf 1 kg neutralisierte lonentauscher-Lösung zugesetzt wurden. Die Dispersion wurde mittels Koaleszierfiter aus Baumwolle, Feinheit 25 µm getrennt. In der lonentauscher-Lösung wurde die Fremdphasenmenge ermittelt, indem nach der Karl-Fischer-Methode der Gesamtwassergehalt der Ionentauscher-Lösung (einschließlich der Reste an wässriger Fremdphase) bestimmt wurde. Die Ergebnisse sind in Tabelle 3 dargestellt.

**Tabelle 3 Gemessene Fremdphasengehalte in der neutralisierten und gewaschenen Ionentauscher-Phase nach Durchströmen des Koaleszlierfilters bei unterschiedlichen Belastungen**

| Durchfluss Dispersion [kg/h cm Kerze] | Fremdphase [Gew.-%] |
|---|---|
| 1,02 | 0,01 |
| 1,86 | 0,05 |
| 3,32 | 0,14 |

Wird für die Phasentrennung kein Koaleszierfilter eingesetzt, beträgt der Wasserphasengehalt in der neutralisierten und gewaschenen lonentauscher-Phase bei einem Leistungseintrag von 4 kW während der Dispergierung nach 10 min. Phasentrennzeit 5 Gew.-%.

## Patentansprüche

1. Verfahren zur Reinigung einer wässrigen Glyoxal-Lösung, die mindestens eine Säure enthält, durch extraktive Säureabtrennung, umfassend die Schritte
i) Vermischen und Dispergieren der wässrigen Glyoxal-Lösung mit einer Ionentauscher-Lösung, enthaltend 20 bis 60 Gew.-% eines tertiären Amins und 80 bis 40 Gew.-% eines mit Wasser nicht beliebig mischbaren organischen Lösungsmittels bei einer Temperatur von 30 bis 100°C,
ii) Phasentrennung und Abtrennung der der mit der Säure beladenen Ionentauscher-Lösung von der wässrigen Glyoxal-Lösung bei Temperaturen von 30 bis 100°C, und
iii) Regeneration der lonentauscher-Lösung durch Inkontaktbringen mit einer basischen Verbindung zur Neutralisation der Säure und Abtrennung der basischen Verbindung,
iv) Vermischen und Dispergieren der lonentauscher-Lösung mit Wasser zur Reinigung von bei der Neutralisation gebildeten Salzen, wobei eine Dispersion aus Wasser und lonentauscher-Lösung erhalten wird,
v) Phasentrennung und Abtrennung der wässrigen Phase von der Ionentauscher-Lösung, und Rückführung der lonentauscher-Lösung in Schritt i),
**dadurch gekennzeichnet, dass** Phasentrennung und Abtrennung der wässrigen Phase in Schritt v) derart erfolgen, dass der Gehalt der wässrigen Phase der in Schritt v) erhaltenen regenerierten lonentauscher-Lösung < 1 Gew.-% beträgt.

2. Verfahren nach Anspruch 1, **dadurch gekennzeichnet, dass** in Schritt i) die Verweilzeit, während der sich die wässrige Glyoxal-Lösung und die lonentauscherlösung in Durchmischung befinden, 1 sec bis 20 sec beträgt.

3. Verfahren nach Anspruch 1 oder 2, **dadurch gekennzeichnet, dass** in Schritt v) die Dispersion aus Wasser und Ionentauscher-Lösung zur Phasentrennung durch einen Koaleszierfilter geleitet wird.

4. Verfahren nach einem der Ansprüche 1 bis 3, **dadurch gekennzeichnet, dass** der Glyoxal-Gehalt der in Schritt i) eingesetzten wässrigen Glyoxal-Lösung 38 bis 45 Gew.-% beträgt.

## Claims

1. A process for purifying an aqueous glyoxal solution comprising at least one acid by extractive acid removal, comprising the steps of
i) mixing and dispersion of the aqueous glyoxal solution with an ion exchanger solution comprising 20 to 60% by weight of a tertiary amine and 80 to 40% by weight of an organic solvent which does not have unlimited miscibility with water at a temperature of 30 to 100°C,
ii) phase separation and removal of the ion exchanger solution laden with the acid from the aqueous glyoxal solution at temperatures of 30 to 100°C, and
iii) regeneration of the ion exchanger solution by contacting with a basic compound for neutralization of the acid and removal of the basic compound,
iv) mixing and dispersion of the ion exchanger solution with water to free it of salts formed in the neutralization to obtain a dispersion of water and ion exchanger solution,
v) phase separation and removal of the aqueous phase from the ion exchanger solution, and recycling of the ion exchanger solution into step i),
which comprises phase separation and removal of the aqueous phase in step v) in such a way that the content of the aqueous phase of the regenerated ion exchanger solution obtained in step v) is < 1% by weight.

2. The process according to claim 1, wherein the residence time during which the aqueous glyoxal solution and the ion exchanger solution are being mixed in step i) is 1 sec to 20 sec.

3. The process according to claim 1 or 2, wherein the dispersion of water and ion exchanger solution in step v) is passed through a coalescence filter for phase separation.

4. The process according to any of claims 1 to 3, wherein the glyoxal content of the aqueous glyoxal solution used in step i) is 38 to 45% by weight.

## Revendications

1. Procédé de purification d'une solution aqueuse de glyoxal qui contient au moins un acide, par séparation de l'acide par extraction, comprenant les étapes suivantes :
i) le mélange et la dispersion de la solution aqueuse de glyoxal avec une solution d'échangeur d'ions, contenant 20 à 60 % en poids d'une amine tertiaire et 80 à 40 % en poids d'un solvant organique non miscible de manière quelconque avec l'eau à une température de 30 à 100 °C,
ii) la séparation des phases et la séparation de la solution d'échangeur d'ions chargée avec l'acide de la solution aqueuse de glyoxal à des températures de 30 à 100°C, et
iii) la régénération de la solution d'échangeur d'ions par mise en contact avec un composé basique pour la neutralisation de l'acide et la séparation du composé basique,
iv) le mélange et la dispersion de la solution d'échangeur d'ions avec de l'eau pour la purification des sels formés lors de la neutralisation, une dispersion d'eau et de solution d'échangeur d'ions étant obtenue,
v) la séparation des phases et la séparation de la phase aqueuse de la solution d'échangeur d'ions, et le recyclage de la solution d'échangeur d'ions dans l'étape i),
**caractérisé en ce que** la séparation des phases et la séparation de la phase aqueuse à l'étape v) ont lieu de sorte que la teneur en phase aqueuse de la solution d'échangeur d'ions régénérée obtenue à l'étape v) soit < 1 % en poids.

2. Procédé selon la revendication 1, **caractérisé en ce qu'**à l'étape i), le temps de séjour pendant lequel la solution aqueuse de glyoxal et la solution d'échangeurs d'ion sont mélangées est de 1 s à 20 s.

3. Procédé selon la revendication 1 ou 2, **caractérisé en ce qu'**à l'étape v), la dispersion d'eau et de solution d'échangeur de chaleur est conduite dans un filtre à coalescence pour la séparation des phases.

4. Procédé selon l'une quelconque des revendications 1 à 3, **caractérisé en ce que** la teneur en glyoxal de la solution aqueuse de glyoxal utilisée à l'étape i) est de 38 à 45 % en poids.
